# EUROPÄISCHE PATENTSCHRIFT

(11) **EP 1 152 053 B1**
(45) Veröffentlichungstag und Bekanntmachung des Hinweises auf die Patenterteilung: **09.03.2016**
(21) Anmeldenummer: 01110308.2
(22) Anmeldetag: 26.04.2001
(51) Int. Cl.: C12M 3/00, C12N 5/00

(54) **Verfahren und Vorrichtung zur Herstellung eines vaskularisierten bioartifiziellen Gewebes**
Method and apparatus for the preparation of a vascularized bioartificial tissue
Procédé et appareil pour la préparation du tissu bioartificiel vascularise

(30) Priorität: 04.05.2000 DE 10021627
(43) Veröffentlichungstag der Anmeldung: 07.11.2001
(73) Patentinhaber: Corlife OHG, 30625 Hannover (DE)
(72) Erfinder: Haverich, Axel, 30916 Isernhagen (DE); Kofidis, Theo, 30559 Hannover (DE)
(74) Vertreter: Lins, Martina

(56) Entgegenhaltungen:
- MARLER ET AL: "Transplantation of cells in matrices for tissue regeneration" ADVANCED DRUG DELIVERY REVIEWS, Bd. 33, 1998, Seiten 165-182, XP002260207
- HOERSTRUP ET AL: "New pulsatile bioreactor for in vitro formation of tissue engineered heart valves" TISSUE ENGINEERING, Bd. 6, Februar 2000 (2000-02), Seiten 75-79, XP001040483 ISSN: 1076-3279
- TAKAHASHI ET AL: "Vascular endothelial growth factor induces activation and subcellular translocation of focal adhesion kinase (p125FAK) in cultured rat cardiac myocytes" CIRCULATION RESEARCH, Bd. 84, 1999, Seiten 1194-1202, XP002260253
- DATABASE MEDLINE [Online] US NATIONAL LIBRARY OF MEDICINE (NLM), BETHESDA, MD, US; Februar 1990 (1990-02) NICOSIA R F ET AL: "Modulation of microvascular growth and morphogenesis by reconstituted basement membrane gel in three-dimensional cultures of rat aorta: a comparative study of angiogenesis in matrigel, collagen, fibrin, and plasma clot." Database accession no. NLM1690206 XP002261257 & IN VITRO CELLULAR & DEVELOPMENTAL BIOLOGY: JOURNAL OF THE TISSUE CULTURE ASSOCIATION. UNITED STATES FEB 1990, Bd. 26, Nr. 2, Februar 1990 (1990-02), Seiten 119-128, ISSN: 0883-8364
- NIKLASON ET AL: "Functional arteries grown in vitro" SCIENCE, Bd. 284, 16. April 1999 (1999-04-16), Seiten 489-493, XP002200891 ISSN: 0036-8075
- BURSAC ET AL: "Three-dimensional environment promotes in vitro differentiation of cardiac myocytes" PROCEEDINGS OF THE FIRST JOINT BMES/EMBS CONFERENCE, OCTOBER 13-16, 1999, ATLANTA, GA, USA, Oktober 1999 (1999-10), Seite 128 XP010357322 ISBN: 0-7803-5674-8
- NICOSIA R F ET AL: "GROWTH OF MICROVESSELS IN SERUM-FREE MATRIX CULTURE OF RAT AORTA. A QUANTITATIVE ASSAY OF ANGIOGENESIS IN VITRO", LABORATORY INVESTIGATION, NATURE PUBLISHING GROUP, THE UNITED STATES AND CANADIAN ACADEMY OF PALHOLOGY, INC, vol. 63, no. 1, 1 July 1990 (1990-07-01), pages 115-122, XP000604191, ISSN: 0023-6837

## Beschreibung

Die Erfindung betrifft ein Verfahren zur Herstellung eines dreidimensionalen, bioartifiziellen Gewebes, welches lebensfähige Zellen in oder auf einer Matrix aufweist und bei welchem Zellen und Matrix zu dem Gewebe oder einer Vorstufe davon kultiviert werden, ein durch dieses Verfahren aus biologischen Materialien erhaltenes vaskularisiertes Gewebe und einen zugehörigen Versuchsreaktor für wissenschaftliche Zwecke sowie zur Erstellung klinisch anwendbarer Gewebe und Organe.

Unter bioartifiziellen Geweben werden dabei solche verstanden, die aus natürlichen, biologischen Materialien in vitro hergestellt werden und die daher keine natürlichen, aber der Natur möglichst getreu nachgebildeten Gewebe sind.

Insbesondere sind das erfindungsgemäße Verfahren und der zugehörige Reaktor auch zur Erzeugung eines bioartifiziellen Herzmuskelgewebes geeignet.

Morbidität und Mortalität in der westlichen Bevölkerung hängen zu großen Teilen mit dem Funktions- und Gewebsverlust des.Herzens zusammen (Herzinsuffizienz). Dies stellt ein Problem von hoher sozioökonomischer Relevanz dar. Immer häufiger sind Bypassoperationen oder Herztransplantationen als ultimative therapeutische Maßnahme notwendig. Transplantationen bringen jedoch Probleme mit sich, beispielsweise durch den Mangel an geeigneten Spenderorganen oder die Belastung der Transplantationspatienten durch die lebenslange Behandlung zur Unterdrückung von Abstoßungsreaktionen mit sich.

Die Transplantationsproblematik betrifft neben Herzen auch andere nicht mehr funktionsfähige Organe, die ersetzt werden müssen.

Aufgrund dessen werden in der medizinischen Forschung große Anstrengungen auf den Gebieten "Gewebeerhalt" einerseits sowie "Gewebe- und Organersatz" andererseits unternommen.

Die Forschungsbemühungen zum "Gewebe- und Organersatz" umfassen die Verwendung und bessere Adaption xenogener Organe sowie auch das Nachzüchten von dreidimensionalen Geweben aus natürlichen Ausgangsmaterialien, um zumindest Teile der Organe ersetzen zu können.

Frühe zelluläre Formen (z.B. embryonale Stammzellen oder Neugeborenenherzzellen sind in der Vergangenheit in das Empfängermyokard injiziert worden (einzeln sowie als Zellhaufen).

Für die Herstellung bzw. zunächst die Simulation von Herzmuskelgeweben aus einfachen biologischen Grundmaterialien sind bereits Konstrukte aus Rattenherzmuskelzellen in Kollagen, angereichert mit Nährstoffen und Wachstumsfaktoren, erzeugt worden, an denen bereits Kontraktionen nachgewiesen werden konnten. Um ein funktionsfähiges dreidimensionales Gewebe, welches für eine Transplantation geeignet wäre und geschädigtes Organgewebe ersetzen könnte, handelte es sich hierbei jedoch noch nicht (R. L. Carrier et al., "Cardiac Tissue Engineering: Cell Seeding, Cultivation Parameters and Tissue Construct Characterization", Biotechn. Bioengin. 64(5), 580-90, 1999).

Die auf diese Weise bisher erhaltenen künstlichen Herzgewebestücke waren nur wenige Millimeter dick und überlebten allenfalls wenige Wochen. Nach einer Implantation waren sie nicht in der Lage, sich in das Empfängergewebe so zu integrieren, dass die Schlagkraft des Empfängergewebes signifikant verbessert wurde (B.S. Kim et al. "Optimizing Seeding and Culture Methods to Engineer Smooth Muscle Tissue on Biodegradable Polymer Matrices", Biotechn. Bioengin. 57(1), 1998; R.K. Li et al. "In vivo Survival and Function of Transplanted Rat Cardiomyocytes" Circ. Res. 1996; 78: 283-288).

Die Aufgabe der Erfindung besteht daher darin, ein Verfahren für die Herstellung eines verbesserten, dreidimensionalen bioartifiziellen Gewebes anzugeben. Insbesondere wird eine Vaskularisierung des Gewebes angestrebt, damit es versorgt und damit lebensfähig erhalten werden kann.

Die Aufgabe wird mit den Merkmalen des Anspruchs 1 gelöst, wobei im Laufe der Gewebekultivierung eine von den Poren oder Öffnungen ausgehende Gefäßsprossung in das Gewebe hinein erfolgt und ein vaskularisiertes bioartifizielles Gewebe erhalten wird.

Dadurch dass das Gewebe bereits während seiner Kultivierung von einem Gefäß durchzogen wird, kann es sich dreidimensional und naturähnlicher ausbilden. Von dem Hauptgefäß geht eine Gefäßsprossung aus, die zu einem vaskularisierten Gewebe führt. Das erzeugte vaskularisierte Gewebe kann bei einer Transplantation an das Gefäßsystem angeschlossen werden und bleibt dadurch ernährt und lebensfähig. Bereits während der Gewebekultur selbst erfolgt eine natürlichere Modellierung der Gewebeverhältnisse; eine Kultur über längere Zeiträume ist möglich, da die Versorgung aller Zellen, auch der in einem Zellverband innenliegenden, besser gewährleistet werden kann. Eine längere Kultur kann beispielsweise vorteilhaft sein, wenn ein besserer Grad der Ausdifferenzierung der Zellen erreicht werden soll. Die Anzucht in dem nachfolgend noch näher beschriebenen Versuchsreaktor erlaubt auch die Schaffung einer für die Organogenese physiologischen Umgebung. (z.B. atmosphärischer Druck bzw. Vakuum, definierte Gaskonzentrationen (Partialdrücke), biochemische Umgebung usw.)

Unter einem bioartifiziellen Gewebe im Sinne der Erfindung wird ein Gewebe verstanden, das nicht als solches beispielsweise operativ aus einem Organismus entnommen wurde, sondern artifiziell mit Hilfe biologischer Materialien nachgebildet wurde. Hierzu werden generell bestimmte Zellen in einen Zellverbund gebracht, bzw. auf eine stabilisierende Unterlage aufgebracht oder in eine Matrix eingebracht. Die Unterlage bzw. Matrix kann selbst wiederum künstlich oder alternativ biologischen Ursprungs sein. Als künstliche Matrizes kommen Polymere in Frage, insbesondere biologisch abbaubare Polymere. Diese können auch schicht- oder netzförmig vorliegen. Als Matrixbildner kommt insbesondere auch Kollagen in Frage. Für den Ursprung der Kollagenmatrix kann ferner auch ein einem Menschen oder Tier entnommenes Gewebestück, welches im Allgemeinen durch chemische und/oder mechanische Methoden azellularisiert wurde, verwendet werden.

Das bioartifizielle Gewebe soll lebensfähige Zellen enthalten, damit mit diesen ein möglichst langes stabiles und lebensfähiges dreidimensionales biologisches Gewebe gebildet wird.

In einer einfachen Ausführungsform können Kollagen und lebensfähige Zellen vermischt und in dieser Mischung weiter kultiviert werden, d.h. mit einer stetig oder in Abständen erneuerten Nährlösung versorgt werden. Verschiedene nachgebildete Gewebe sind bereits verwendet und vorgeschlagen worden. Der Aufbau dieser Gewebe hängt im Einzelnen von der Art des Gewebes oder auch Organs ab.

Die Hauptproblematik aus biologisch abbaubaren Matrizen und Zellen bestehender Gewebe, insbesondere auch künstlich nachgebildeter Herzgewebe ist ihre limitierte Dimension und Überlebensfähigkeit, denn es war bislang nicht möglich, sie naturähnlich von Blut oder Nährmedium ernähren zu lassen (siehe N Bursac et al. "Cardiac Muscle Tissue Engineering: toward an in vitro an in vivo model for electrophysiological studies" Am J Physiol. 277 (2), HH433-H444, August 1999; R.K. Li et al. "Survival and Function of Bioengineered Cardiac Grafts", Circulation 1999, 100 [suppl. II];II-63-II-69).

Der Erfindung liegt daher die Erkenntnis zugrunde, dass die Fähigkeit eines Gewebes zu überleben und seine Funktionen zu entfalten, maßgeblich von seiner Vaskularisierung (dem Gefäßvorkommen innerhalb des Gewebes) abhängig ist. Das erfindungsgemäße Verfahren sieht daher vor, dass in das künstliche biologische Gewebe zu Beginn seiner Herstellung wenigstens ein Gefäß eingesetzt wird, welches von außen versorgt wird. Durch die Versorgung des Gefäßes mit geeigneter Nährlösung oder Blut erfolgt im Laufe der Kultivierung eine natürliche Gefäßsprossung in das Gewebe hinein.

Das Gefäß kann in die Matrix, beispielsweise eine azellularisierte Kollagenmatrix, vor der Besieldung mit den für das künstliche biologische Gewebe gewünschten Zellen eingesetzt werden oder es kann, wenn die Herstellung des künstlichen Gewebes lediglich ein Vermischen von Zellen und Matrixmaterial umfasst, in diese Mischung zu Beginn der Kultur eingelegt werden.

Als Gefäß kann ein solches natürlichen Ursprungs von Mensch oder Tier verwendet werden, beispielsweise - insbesondere für wissenschaftliche Versuche - Rattenaorten. Es kann aber auch ein künstliches Gefäß, insbesondere aus einem biologisch kompatiblen Polymer eingesetzt werden; es kann auch der gesamte Gefäßbau eines nachzureichenden Organes eingesetzt werden.

Vorzugsweise wird ein natives großlumiges, möglichst artspezifisches Gefäß verwendet. Das langgestreckte Gefäßlumen kann seitlich abgehend Öffnungen oder Seitenäste aufweisen. Die Öffnungen entstehen im einfachsten Fall dadurch, dass von dem Hauptast eines nativen Gefäßes Seitenäste abgeschnitten werden. Von diesen Öffnungen ausgehend wird bei der Kultur des künstlichen Gewebes eine Gefäßsprossung in das Gewebe hinein erfolgen.

Alternativ kann auch ein in sich poröses Gefäß verwendet werden. Die Porosität erleichtert den Beginn der Gefäßsprossung und könnte durch eine chemische und/oder mechanische Behandlung des Gefäßes erzielt werden. Die mechanische Behandlung kann auch in einer wiederholten Druckbelastung des Gefäßes bestehen, beispielsweise indem das Gefäß von einem pulsativen Fluss durchströmt wird. Falls ein künstliches Gefäß verwendet wird, erscheinen tubuläre Fasernetze besonders geeignet. Die Porosität des künstlichen Materials kann mit technischen Mitteln vorab beliebig eingestellt werden. Es kann beispielsweise ein gewebeartiges Kunststoffmaterial mit frei wählbarer Porosität verwendet werden. Das in sich poröse Gefäß kann punktuell mit einem einen Anreiz für die Gefäßsprossung auslösenden Stoff oder Mittel präpariert werden, beispielsweise dem Protein VEGF (vascular endothelial growth factor).

Für das Verfahren ist weiterhin wesentlich, dass das Gefäß von innen oder von außen mit Blut oder einem Kulturmedium versorgt wird. Hierfür kann das Gefäß in üblicher Weise perfundiert werden, d.h. es kann an einen Kreislauf von Blut oder Kulturmedium angeschlossen sein. Dem Kulturmedium können die Angiogenese fördernden Stoffe, wie VEGF oder andere bekannte Wachstumsfaktoren (TGF, PDGF, VGF) zugesetzt werden; solche Stoffe und insbesondere Wachstumsfaktoren können auch der Gewebematrix oder allgemein der Gewebemischung direkt zugesetzt werden.

Die Perfusion kann vorzugsweise unter Druck erfolgen. Im einfachsten Falle ist hierfür vorgesehen, dass die Perfusionszuleitung einen größeren Durchmesser als die Perfusionsableitung hat, so dass sich innerhalb des Gefäßes ein Druck aufbaut. Ein solcher Druck wird auch in der Natur in jedem Organ aufrechterhalten, so dass die Perfusion unter Druck zur Simulation natürlicher Verhältnisse beiträgt. Durch den Druck wird Flüssigkeit ins Gewebe abgepresst und es entsteht ein mechanischer Reiz, der die Gefäßsprossung fördert. Die Perfusion unter Druck kann auch auf andere Weise mit geeigneten Pumpen und Drosselelementen bewerkstelligt werden.

Als besonders vorteilhaft wird es angesehen, wenn die Perfusion pulsatil erfolgt, da dies einer natürlichen, mit dem Herzschlag pulsierenden Versorgung des Gewebes am nächsten kommt.

Die technische Realisierung einer pulsatilen Perfusion, unter mehr oder weniger Druck, ist grundsätzlich im Stand der Technik bekannt und muss daher hier nicht näher beschrieben werden.

Das Gefäß kann durch die für die Perfusion erforderlichen Zu- und Ableitungen auch innerhalb des Gewebes positioniert und fixiert werden. Dies gilt insbesondere für Gewebekulfuren aus gelartigen Mischungen.

In einer bevorzugten Ausführungsform werden als Zellen innerhalb des künstlichen zu vaskularisierenden Gewebes Kardiomyozyten verwendet, so dass sich ein künstliches Herzgewebe ergibt. Das Prinzip der Gefäßsprossung ist jedoch auch auf andere bioartifizielle Gewebe anwendbar, beispielsweise auf Hautgewebe. Im letzteren Fall werden als Zellen vorzugsweise Keratinozyten verwendet.

In entsprechender Weise können Nieren- Lungen- und andere Gewebe erhalten werden. Durch Vorgeben der geometrischen Form der Kultur und Einsetzen eines Gefäßbaums für ein ganzes Organ können ganze bioartifizielle Organe (Herz, Niere, Lunge) erhalten werden.

Durch das erfindungsgemäße Verfahren ist es erstmals möglich, ein vaskularisiertes, bioartifizielles, dreidimensionales Gewebe zu erhalten, welches sich dadurch auszeichnet, dass es von wenigstens einem Gefäß natürlichen Ursprungs von Mensch oder Tier oder wenigstens einem biokompatiblen, künstlichen Gefäß durchzogen ist, wobei von dem Gefäß weitere in das Gewebe gesprosste Gefäße abgehen. In einer bevorzugten Ausführungsform ist das vaskularisierte, bioartifizielle Gewebe ein künstliches Herzgewebe, welches Kardiomyozyten in einer Matrix aufweist. In einer weiteren bevorzugten Ausführungsform ist das vaskularisierte, bioartifizielle Gewebe ein künstliches Hautgewebe, das Keratinozyten in einer Matrix aufweist.

Die vaskularisierten Gewebe sind als Transplantate besser in den Empfängerkörper integrierbar, da sie von Beginn an durchgehend versorgt werden können. Bei der Verwendung der Gewebe als Transplantate können die Gefäße des künstlichen biologischen Gewebes mit dem körpereigenen Gefäßsystem des Empfängers verbunden werden, so dass eine sofortige Versorgung des neu eingesetzten Gewebes gesichert ist. Für künstliche Herzgewebe ergeben sich insbesondere die Vorteile, dass die Gewebestücke erstmals eine Dicke von wenigen mm überschreiten können und dass die Überlebensfähigkeit des vaskularisierten, dann dreidimensionalen Gewebes die eines nichtvaskularisierten Gewebes deutlich übersteigt.

Die Erfindung umfasst weiterhin den Versuchsreaktor gemäß Anspruch 17 für die kontrollierte Herstellung eines bioartifiziellen Gewebes nach dem erfindungsgemäßen Verfahren zur Herstellung eines vaskularisierten künstlichen Gewebes. Dieser Versuchsreaktor besitzt wenigstens eine Gewebekultur-Kammer, wenigstens einen Zu- und Ablauf zu dieser bzw. jeder Kammer, wobei wenigstens ein Zu- und ein Ablauf als Mittel zur Halterung eines Gefäßes ausgebildet ist, sowie zwei sich gegenüberliegende, plane, parallele und optisch transparente Begrenzungswände dieser bzw. jeder Gewebekulturkammer. Der Versuchsreaktor soll insbesondere dazu dienen, die biologisch-physiologische Erforschung bioartifizieller Gewebe zu erleichtern. Hierzu ist der Reaktor so ausgebildet, dass der Inhalt der Kulturkammer mit optischen Verfahren untersucht und beobachtet werden kann. Durch die wenigstens zwei sich gegenüberliegenden planen parallelen und optisch transparenten Begrenzungswände kann der Reaktor während des Kulturprozesses unmittelbar optischen Untersuchungsmethoden ausgesetzt werden, der Inhalt der Reaktor-Gewebekulturkammer(n) kann direkt mit einem Mikroskop betrachtet, der Inhalt kann fotografiert oder gefilmt werden.

Im Falle mehrerer getrennter Gewebekulturkammern können Reihenuntersuchungen unter Variation bestimmter Parameter durchgeführt werden, wodurch ebenfalls die Erforschung bioartifizieller Gewebe deutlich systematisiert und erleichtert wird. Innerhalb des vorgeschlagenen Versuchsreaktors sind mannigfaltige Möglichkeiten gegeben, auf den Kulturprozess Einfluss zu nehmen und verschiedene wachstumsfördernde Maßnahmen zu ergreifen. Die Blut- und Nährstoffversorgung des bioartifiziellen Gewebes kann gut beobachtet und durch die Variation verschiedener Parameter optimiert werden.

Dabei ist der Versuchsreaktor einfach aufgebaut und kostengünstig herzustellen.

Der Versuchsreaktor ist vorzugsweise pro Kammer mit wenigstens einem absperrbaren Zu- und Ablauf versehen, wobei die Absperrmittel vorzugsweise Hähne, Ventile, Schlauchklemmen und/oder Diaphragmen umfassen. Die Zu- und Abläufe dienen gleichzeitig dazu, die nach dem erfindungsgemäßen Verfahren innerhalb der Gewebekultur zu positionierenden Gefäße festzulegen und zu fixieren. Es können gesonderte Mittel zur Fixierung der Gefäße vorhanden sein, ebenso wie Mittel zur Fixierung einer extrazellulären Matrix, wie beispielsweise eines azellularisierten Kollagensubstrats.

Erfindungsgemäß ist eine der planen optisch transparenten Begrenzungswände der Kammer als Deckel ausgebildet. Für eine Einflussnahme auf das Kulturmedium oder das Substrat ist es vorteilhaft, wenigstens einen zusätzlichen absperrbaren Zulauf, bzw. eine Zugabestelle an jeder Kammer vorzusehen. Hierfür können beispielsweise Stutzen mit Schraubansätzen dienen, die mit in Schraubringe eingesetzten Diaphragmen versehen sind. Durch diese Zugabestellen können dann mittels einer Spritze bestimmte Substanzen zugefügt werden, die auf die Entwicklung des bioartifiziellen Gewebes in der Kultur Einfluss nehmen können (siehe oben, z.B. VEGF).

Der Zulauf oder die Zuläufe des Reaktors sind vorzugsweise mit einer Mikropumpe und einem Schlauchsystem verbunden, durch welches Blut oder Nährmedium zugeführt wird. Die Ableitung oder Ableitungen sind vorzugsweise mit einem Abfallbehälter verbunden. Im folgenden wird die Erfindung anhand von Zeichnungen näher erläutert, die eine Ausführungsform des erfindungsgemäßen Versuchsreaktors zeigen und anhand derer auch ein Ausführungsbeispiel für die Durchführung des Verfahrens zur Herstellung eines bioartifiziellen Herzgewebes erläutert werden wird.
Abb. 1 ist eine schematische Darstellung eines Ausführungsbeispiels des Versuchsreaktors mit zehn parallel geschalteten Gewebekulturkammern, in perspektivischer Darstellung (a), in Draufsicht von der Schmalseite (b), in Draufsicht von oben (c), sowie von oben im Detail (d);
Abb. 2 zeigt eine vereinfachte schematische Darstellung des in Figur 1 gezeigten Reaktors mit den Anschlussmöglichkeiten für Zu- und Abläufe;
Abb. 3 zeigt schematisch die Versorgung für die Zu- und Abläufe des in Figuren 1 und 2 dargestellten Ausführungsbeispiels;
Abb. 4 ist eine schematische Darstellung einer einzelnen Gewebekulturkammer mit einem darin eingespannten Gefäß,
Abb. 5 zeigt die Ausbildung eines Versuchsreaktors aus zehn Gewebekulturkammern mit einem gemeinsamen Deckel in perspektivischer Ansicht;
Abb. 6 zeigt das Ausführungsbeispiel nach Figur 5 mit einem anderen. Deckel mit zusätzlichen mit Diaphragmen verschlossenen Zugabestellen.

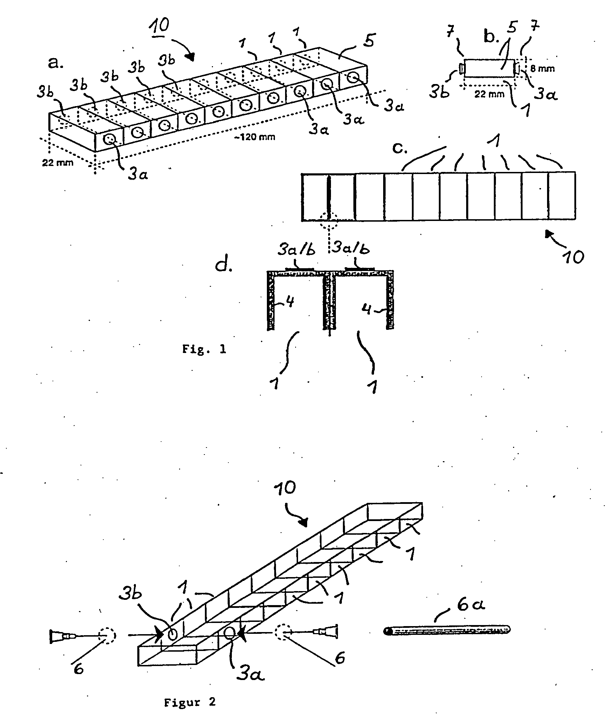

Abb. 1 zeigt einen im Ganzen mit zehn bezeichneten Versuchsreaktor aus zehn parallel geschalteten quaderförmigen Gewebekulturkammern 1

Der gesamte Reaktor 10 ist blockförmig ausgelegt und besteht im vorliegendem Ausführungsbeispiel aus Glas. Die Gesamtlänge der zehn Gewebekulturkammern beträgt hier ca. 120 mm, die Breite 22 mm, die Höhe 8mm. Die Breite von 22 mm entspricht der Länge einer einzelnen Gewebekulturkammer, die über diese Länge vom Zulauf 3a aus bis zum Ablauf 3b durchflossen wird. Die Seitenansicht der einzelnen Gewebekulturkammern, bzw. die Frontansicht des Reaktors 10 insgesamt, ist in Abbildung b nochmals genauer dargestellt. Die Figur 1c zeigt eine Draufsicht auf den blockförmigen Reaktor mit den einzelnen Kammern 1. Ein Detail dieser Abbildung ist nochmals in Figur 1d gezeigt, aus der zu ersehen ist, wie die einzelnen Kammern mit einer Silikonauskleidung 4 versehen sind. In diesem Beispiel sind die Innenflächen der Kammern 1 sowie das Innere der Zu- und Abläufe 3a, 3b mit Silikon ausgekleidet, das biokompatibel ist und mit dem Gewebe nicht verklebt.

Für die Beobachtbarkeit des Substrates während der Kultur ist es von entscheidender Bedeutung, dass die oberen und unteren Begrenzungswände 5 der Kammern, d.h. heißt Deckel und Boden des Reaktors 10, sich plan und parallel gegenüberliegen und aus einem optisch transparenten Material wie hier Glas bebildet sind. Die Seitenwände es Reaktors könnten demgegenüber auch aus Kunststoff, beispielsweise direkt aus Silikon bestehen. Diese erfindungsgemäße Ausgestaltung mit den planen Begrenzungswänden 5 ermöglicht die Durchführung optischer Untersuchungen direkt am Reaktor während der Kultur, z.B. eine Beobachtung durch Mikroskop, Fotografie oder Filmen.

Abb. 2 zeigt einen entsprechenden Versuchsreaktor 10 mit wiederum 10 Gewebekulturkammern 1 in einer vereinfachten schematischen Darstellung. Es sind daher hier nicht alle Zu- und Abläufe 3a, 3b gezeigt. Die Zu- und Abläufe der einzelnen Gewebekammern 3a und 3b - hier vereinfacht für nur eine der Kammern andeutungsweise dargestellt - sind im vorliegenden Ausführungsbeispiele mit Silikon abgedichtet, sie könnten auch mit üblichen Diaphragmen verschlossen sein. Durch die Pfeile in Figur 2 wird angedeutet, wie in diese Dichtungen übliche Spritzenköpfe 6 eingeführt werden, wobei diese Spritzenköpfe 6 mit Schlauchleitungssystemen zur Zu- und Ableitung von Nährlösungen bzw. Blut verbunden sind. Als Detail zeigt 6a eine abgeschliffene Kanülenspitze wie sie vorteilhaft zur Zu- und Abführung von Medium verwendet werden kann. Dabei werden vorzugsweise Kanülen verschiedener Dicke eingesetzt, so dass hierdurch wie oben bereits beschrieben ein Druck in dem zwischen den Kanülen liegenden Gefäß aufgebaut werden kann, wenn die Zuleitung über die gröbere Kanüle, die Ableitung jedoch über die feinere Kanüle erfolgt. (Stärken 18G, 21G in diesem Beispiel).

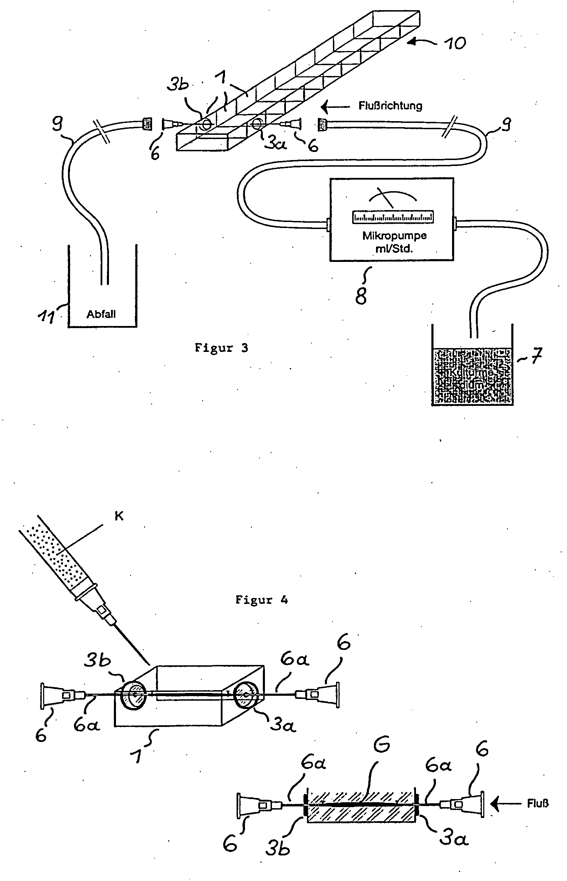

Abb. 3 zeigt das entsprechende Ausführungsbeispiel, wobei die Spritzenköpfe 6 mit den Kanülenpitzen durch die Silikondichtungen der Zu- und Ableitungen 3a, 3b geführt sind. Aus Gründen der zeichnerischen Vereinfachung ist nur eine Gewebekulturkammer mit den Zu- und Ableitungen dargestellt, die übrigen Verbindungen wurden nur in der Zeichnung weggelassen. In dem dargestellten Ausführungsbeispiel sind alle Gewebekulturkammern 1 parallel auf die gleiche Weise mit dem Kulturmedium-Vorratsbehälter 7, der Pumpe 8, dem Schlauchsystem 9 und dem Abfallbehälter 11 verbunden.

Abb. 4 zeigt einen Längsschnitt durch eine einzelne Gewebekammer längs eines zwischen Zu- und Ablauf 3a, 3b eingespannten Gefäßes G. Entsprechende Teile sind mit entsprechenden Bezugszeichen gekennzeichnet. Das Gefäß G wird über die Kanülen 6a der Spritzenköpfe 6 gezogen, die durch die Dichtungen der Zu- und Abläufe 3a,3b geführt sind, und wird auf diese Weise sowohl gehaltert als auch innerhalb der Gewebekulturkammer positioniert, dass es sich etwa mittig in der die Kammer ausfüllende Mischung aus Zellen und Matrigel befindet. Die Perfusion erfolgt wieder wie in Figur 3 gezeigt.

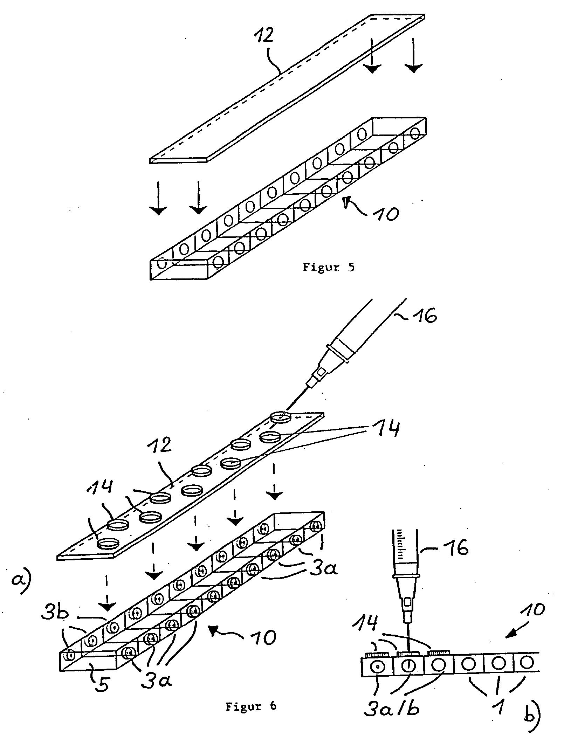

Ebenfall in Abb. 4 gezeigt ist eine perspektivische Ansicht derselben Kammer wobei diese ähnlich wie eine Petrischale oben offen ist und mit einer einfachen Spritze mit der Mischung K der Ausgangsstoffe für das künstliche Gewebe beschickt werden kann. Die Kammer kann dann anschließend mit einem Deckel verschlossen werden wie in Figur 5 noch gezeigt werden wird.

Abb. 5 zeigt den Versuchsreaktor 10 in der gleichen Ausführungsform wie in Abbn. 1 bis 4 jedoch in vereinfachter schematischer Darstellung in einer Explosionsdarstellung, aus der hervorgeht, wie der blockförmige Reaktorkörper aus den zehn einzelnen Gewebekulturkammern 1 mit einem abnehmbaren Deckel 12 verbunden werden kann. Während die Kultur je nach dem gewünschten Gewebe auch in einem offenen Reaktor durchgeführt werden könnte - ähnlich wie in einer Petrischale - ist der Deckel 12 als Mikroskopier- und Transportdeckel vorgesehen. Generell schützt der Deckel die Kultur vor Verunreinigungen. Der Deckel 12 ist zu dem Boden des Reaktors 10 parallel; beide Flächen sind plan und optisch und transparent, so dass spektroskopische Untersuchungen, das Fotografieren, das Mikroskopieren oder einfache Betrachten der Kultur innerhalb dieses geschlossenen geschützten Behälters direkt erfolgen kann.

Abb. 6 zeigt den Reaktor des bisher besprochenen Ausführungsbeispiels mit zehn einzelnen Kulturkammern in einer Explosionsdarstellung mit einem anderen Deckel 12 (Kulturdeckel), der jeweils oberhalb der einzelnen Kammern mit Schraubansätzen versehen ist, die mit in Schraubringen gehalterten Diaphragmen 14 verschlossen sind. Durch diese zusätzlichen Zuläufe bzw. Zugabestellen können den einzelnen Gewebekulturkammern 1 getrennt bestimmte Substanzen wie Wachstumsfaktoren oder Medienzusätze zugeführt werden, so dass eine gezielte Variation der Kulturbedingungen innerhalb eines Reihenexperimentes in dem Reaktor 10 möglich ist. Abb. 6a zeigt eine perspektivische Explosionsdarstellung dieses Reaktors. Abb. 6b zeigt denselben Reaktor in geschlossenem Zustand in einer Seitenansicht. Durch eine Spritze 16 soll angedeutet werden, wie den einzelnen Gewebekulturkammern 1 bestimmte Substanzen zugeführt werden können.

Der erfindungsgemäße Versuchsreaktor besitzt vor allem die folgenden Vorteile:
1. Er ist der erste Bioreaktor der als Herzmuskelbioreaktor verwendbar ist und nicht nur als Kulturkammer dient sondern gleichzeitig natürliche dynamische Prozesse, wie die Blutversorgung durch elastische pulsierende Gefäße immitieren und Druckveränderungen umsetzen kann. Diese Druckveränderungen kommen ebenfalls im lebenden Organismen vor und haben entscheidenden Einfluss auf die Entwicklung der Gewebekultur (Fluss, Druckgradient, Osmose, Diapedese, Scheerstress).
2. Er ist der erste Bioreaktor, der auf derartig einfache Weise unter sterilen Bedingungen vom Betrieb genommen und auf verschiedene Weise manipuliert werden kann. Besonders vorteilhaft ist, dass der Reaktor durch seine planen und parallelen Begrenzungsflächen auf ein herkömmliches Mikroskop platziert und das Kultivierungsexperiment in jeder Zwischenphase dokumentiert werden kann.
3. Er erlaubt durch die zusätzlichen Zugabestellen, die erfindungsgemäßim Deckel angeordnet sind, zahlreiche Applikationen verschiedener Substanzen während jeder Phase des Experimentes.
4. Die Ausbildung des Versuchsreaktors in mehreren Kammern ermöglicht modulmäßig Vergleichsstudien innerhalb einer Versuchsreihe, bei der bestimmte Parameter gleich gehalten werden können. Hierdurch kann der Arbeitsaufwand für Versuchsreihen beträchtlich reduziert werden, so dass sich die Arbeitsgeschwindigkeit insgesamt erhöht. Der Reaktor ermöglicht die Durchführung von Versuchsreihen mit vielen Freiheitsgraden und Variablen. Es können durch die einzelnen Kammern unterschiedliche Medien gepumpt werden, die Zusammensetzung eines Grundmediums kann in den verschiedenen Kammern variiert werden, es können bestimmte Substanzen nur einzelnen Kammern zugeführt werden oder es kann über die Anzahl der Kammern ein Konzentrationsgradient derselben Substanz aufgegeben werden. Die Perfusion der Gefäße in den Kulturkammern kann physikalisch in breitem Rahmen variiert werden, z.B. durch pulsatilen oder nicht-pulsatilen Fluss und durch Variation der Flussrate. Schließlich kann eine zusätzlich elektrische Anregung der Zellen durch eine elektrische Stimulation der Kultur über elektrische Anschlüsse in den einzelnen Kammern erfolgen.
5. Die flache Bauweise und parallele Schaltung der Kammern macht es möglich, dass mehrere Versuchsreaktoren übereinander aufgestockt werden können, so dass aus mehreren Modulen eine komplexe Kultur- und Perfusionsanlage gebaut werden kann.
6. Größe und Bauprinzip sind so gestaltet, dass der Konstruktionsaufwand klein bleibt, damit der Reaktor schnell angebaut und in Betrieb genommen werden kann.

### Verfahrensbeispiel für eine wissenschaftliche Versuchsreihe

Es wurden neonatale Rattenmuskelherzen aus eigener Zucht und durch etablierte enzymatische Methoden Zellen daraus gewonnen. Während die Zellen für die Kultur vorbereitet werden, werden innerhalb des Versuchsreaktors frisch gewonnene Rattenaorten zwischen den Kanülen eingespannt und die Kanülen mit einer Mikropumpe verbunden. Dazwischen wird seriell ein Mediumreservoir geschaltet. Nach Vorbereitung und Reinigung der Zellen werden diese in Matrigel gegossen und das Gemisch in jede einzelne Kammer eingefüllt. Die Pumpe wird zwei Stunden später eingeschaltet, nachdem das Gel-Zell-Gemisch solide geworden ist. Durch die Löcher der abgeschnittenen Aortenäste erhält das Gemisch ständigen mechanischen, ernährenden und migrativen (Zellwanderung auslösenden) Reiz. Dies veranlasst die Aussprossung mikrotubulärer Strukturen ausgehend von der eingespannten Aorta in das Gemisch, die man unter dem Mikroskop beobachten kann. In einer ersten Versuchsreihe wurde Kulturmedium mit einem 5%-igen 02-Gehalt durch das Gefäß gepumpt. Eine Stunde nach Beginn der Perfusion verabreicht man durch die Deckeldiaphragmen das Protein VEGF- einen die Gefäßsprossung fördernden Faktor in die Hälfte der Kammern. Den Rest der Kammern perfundiert man nur. Die VEGF-Applikation wiederholt sich täglich.

Nach 7 Tagen Perfusion entnimmt man das solide Gewebe samt Gefäß, man fixiert es in Glutaraldehyd und man führt es in mehreren Schnitten der HE- sowie immunhistochemischen Färbung zum Nachweis von Troponin, Tropomyosin, aber auch der Messung der Lactat- und Glucosekonzentration in variablen Abständen vom zentral verlaufenden Aortengefäss zu. Man vergleicht einfach perfundierte mit durch Wachstumsfaktor behandelten EHTs bezüglich der Zellvitalität, Stoffwechselaktivität und Gefäßreichtum. Im nächsten Versuchsschritt kann ein weiterer Gefäßsprossungsstimulator erprobt und die 02-Konzentration verändert werden. Die Fixierung erfolgt jeweils nach verschieden langen Zeitintervallen, um die Parameter zu identifizieren, die die bestmögliche Zellvitalität und das höchste Gefäßvorkommen bewirken.

## Patentansprüche

1. Verfahren zur Herstellung eines dreidimensionalen bioartifiziellen Gewebes, welches lebensfähige Zellen in oder auf einer Matrix aufweist und bei welchem Zellen und Matrix zu dem Gewebe oder einer Vorstufe davon kultiviert werden, **dadurch gekennzeichnet, dass** in das Gewebe zu Beginn seiner Herstellung wenigstens ein poröses Gefäß oder ein Gefäß, das von einem lang gestreckten Gefäßlumen seitlich abgehende Öffnungen aufweist, eingesetzt wird, welches durch Perfusion des Gefäßes von außen versorgt wird, indem es mit Blut oder einem Kulturmedium perfundiert wird, so dass im Laufe der Gewebekultivierung eine von den Poren oder Öffnungen ausgehende Gefäßsprossung in das Gewebe hinein erfolgt und ein vaskularisiertes bioartifizielles Gewebe erhalten wird.

2. Verfahren nach Anspruch 1, **dadurch gekennzeichnet, dass** als Gefäß ein solches natürlichen Ursprungs von Mensch oder Tier verwendet wird.

3. Verfahren nach Anspruch 1, **dadurch gekennzeichnet, dass** als Gefäß ein künstliches Gefäß verwendet wird.

4. Verfahren nach Anspruch 3, **dadurch gekennzeichnet, dass** das künstliches Gefäß aus einem ein biologisch kompatiblen Polymer besteht.

5. Verfahren nach einem der Ansprüche 1 bis 4, **dadurch gekennzeichnet, dass** das poröse Gefäß punktuell mit einem einen Anreiz für die Gefäßsprossung auslösenden Stoff oder Mittel präpariert wurde.

6. Verfahren nach einem der Ansprüche 1 bis 5, **dadurch gekennzeichnet, dass** dem Kulturmedium die Angiogenese fördernde Stoffe zugesetzt werden.

7. Verfahren nach Anspruch 6, **dadurch gekennzeichnet, dass** dem Kulturmedium Wachstumsfaktoren zugesetzt werden.

8. Verfahren nach Anspruch 7, **dadurch gekennzeichnet, dass** dem Kulturmedium als ein Wachstumsfaktor VEGF (vascular endothelial growth factor) zugesetzt wird.

9. Verfahren nach einem der Ansprüche 1 bis 8, **dadurch gekennzeichnet, dass** die Perfusion unter Druck erfolgt.

10. Verfahren nach einem der Ansprüche 1 bis 9, **dadurch gekennzeichnet, dass** die Perfusion pulsatil erfolgt.

11. Verfahren nach einem der Ansprüche 1 bis 10, **dadurch gekennzeichnet, dass** das Gefäß so zwischen die für die Perfusion erforderlichen Zu- und Ableitungen eingespannt wird, dass es gleichzeitig relativ zu dem zu kultivierenden Gewebe positioniert und fixiert wird.

12. Verfahren nach einem der Ansprüche 1 bis 11, **dadurch gekennzeichnet, dass** als Zellen innerhalb des zu vaskularisierenden, dreidimensionalen Gewebes Kardiomyozyten verwendet werden und das erhaltene Gewebe ein bioartifizielles Herzgewebe ist.

13. Verfahren nach einem der Ansprüche 1 bis 12, **dadurch gekennzeichnet, dass** als Zellen Keratinozyten verwendet werden und das erhaltene Gewebe ein bioartifizielles Hautgewebe ist.

14. Vaskularisiertes bioartifizielles Gewebe erhalten nach einem der Ansprüche 1 bis 13, **dadurch gekennzeichnet, dass** es von wenigstens einem Gefäß natürlichen Ursprungs von Mensch oder Tier oder wenigstens einem biokompatiblen künstlichen Gefäß durchzogen ist, wobei von dem Gefäß weitere in das Gewebe gesprosste Gefäße abgehen.

15. Vasularisiertes bioartifizielles Gewebe nach Anspruch 14, **dadurch gekennzeichnet, dass** das Gewebe ein künstliches Herzgewebe ist, welches Kardiomyozyten in einer Matrix aufweist.

16. Vaskularisiertes bioartifizielles Gewebe nach Anspruch 14, **dadurch gekennzeichnet, dass** das Gewebe ein künstliches Hautgewebe ist, welches Keratinozyten in einer Matrix aufweist.

17. Versuchsreaktor für die kontrollierte Herstellung eines bioartifiziellen Gewebes nach einem Verfahren gemäß einem der Ansprüche 1 bis 13, **gekennzeichnet durch** wenigstens eine Gewebekultur-Kammer, wenigstens einen Zu- und Ablauf zu dieser/jeder Kammer, wobei wenigstens ein Zu- und ein Ablauf als Mittel zur Halterung eines Gefäßes ausgebildet ist, sowie zwei sich gegenüberliegende, plane, parallele und optisch transparente Begrenzungswände dieser/jeder Gewebekultur-Kammer, wobei eine der planen optisch transparenten Begrenzungswände der Kammer als Deckel ausgebildet ist und in dem Deckel zusätzliche Zugabestellen oberhalb jeder Kammer angeordnet sind.

18. Versuchsreaktor nach Anspruch 17, **dadurch gekennzeichnet, dass** pro Kammer wenigstens je ein absperrbarer Zu- und Ablauf vorhanden ist.

19. Versuchsreaktor nach Anspruch 18, **dadurch gekennzeichnet, dass** Absperrmittel der absperrbaren Zu- und Abläufe Hähne, Ventile, Schlauchklemmen und/oder Diaphragmen umfassen.

20. Versuchsreaktor nach einem der Ansprüche 17 bis 19, **dadurch gekennzeichnet, dass** in wenigstens einer Gewebekultur-Kammer elektrische Anschlüsse und/oder elektrische Bauteile vorgesehen sind.

## Claims

1. Process for producing a three dimensional bioartificial tissue having viable cells in or on a matrix and with which the cells and matrix can be cultivated to a tissue or precursor of it, **characterized in that** at the beginning of the production at least one vessel or porous vessel wherein the vessel has lateral openings departing from a longitudinally extended vessel lumen is inserted into the tissue whereby the perfusion of the vessel is from outside by perfusion with blood or a culture medium and cultivating said tissue in a manner of hereby vessels propagate into the tissue and a vascularized bioartificial tissue obtained.

2. The method according to claim 1, **characterized in that** the vessel is of natural origin selected from the group consisting of humans and animals.

3. The method according to claim 1, **characterized in that** the vessel is a synthetic vessel.

4. The method according to claim 3, **characterized in that** the synthetic vessel consists of a biological acceptable polymer.

5. The method according to any one of claims 1 to 4, **characterized in that** the porous vessel is a vessel whereby the porous vessel is prepared punctually with a substance or means initiating a stimulus for vascular propagation.

6. The method according to any one of claims 1 to 5, **characterized in that** the culture medium is supplied with angiogenesis propagating substances.

7. The method according to claim 6, **characterized in that** the culture medium is supplied with growth factors.

8. The method according to claim 7, **characterized in that** the culture medium is supplied with the growth factor VEGF (vascular endothelial growth factor).

9. The method according to any one of claims 1 to 8, **characterized in that** perfusion is accomplished under pressure.

10. The method according to any one of claims 1 to 9, **characterized in that** the perfusion is accomplished in pulses.

11. The method according to any one of claims 1 to 10, **characterized in that** the vessel is held between inlet and outlet lines needed for the perfusion so that it is simultaneously positioned and fixed in relation to the tissue being cultivated.

12. The method according to any one of claims 1 to 11, **characterized in that** cardiomyocytes are used as cells within the three dimensional tissue to be vascularized and the tissue obtained is a bioartificial heart tissue.

13. The method according to any one of claims 1 to 12 **characterized in that** keratinocytes are used as the cells and the tissue obtained is a bioartificial skin tissue.

14. Vascularized bioartificial tissue obtainable by any one of claims 1 to 13 **characterized in that** at least one vessel passes through said tissue from which other vessels are propagated into the tissue whereby the vessels are of natural origins selected from the group consisting of humans and animals.

15. Vascularized bioartificial tissue according to claim 14, **characterized in that** the tissue is a synthetic heart tissue having cardiomyocytes in a matrix.

16. Vascularized bioartificial tissue according to claim 14, **characterized in that** the tissue is a synthetic skin tissue having keratinocytes in a matrix.

17. Experimental reactor for controlled production of a bioartificial tissue according to a method of any one of claims 1 to 13, **characterized in that** at least one tissue culture chamber; at least one inlet and outlet for said tissue culture chamber, whereby at least one inlet and outlet are designed as means for holding a vessel, as well as two opposite, plane, parallel and optically transparent boundary walls for said at least one tissue culture chamber, whereby at least one of the plane optically transparent boundary walls of the chamber is designed as a lit and wherein additional input points above each chamber of said lit are arranged,

18. Experimental reactor according to claim 17, **characterized in that** for each chamber at least one closable inlet and outlet is present.

19. Experimental reactor according to claim 18, **characterized in that** the closing means of the closable inlet and outlet are cocks, valves, hose clamps and/or diaphragms.

20. Experimental reactor according to any one of claims 17 to 19, **characterized in that** electrical connections and/or electrical components are provided in said at least one tissue culture chamber.

## Revendications

1. Procédé de fabrication d'un tissu bio-artificiel bidimensionnel, qui comprend des cellules viables dans ou sur une matrice, et dans lequel des cellules et une matrice sont cultivées pour donner le tissu ou un précurseur de ce dernier, **caractérisé en ce qu'**on introduit dans le tissu, au début de sa fabrication, au moins un vaisseau poreux ou un vaisseau qui présente des ouvertures partant latéralement à partir d'une lumière vasculaire s'étendant longitudinalement, vaisseau qui est alimenté de l'extérieur par perfusion du vaisseau, par perfusion de sang ou d'un milieu de culture de telle sorte que, au cours de la culture du tissu, un bourgeonnement vasculaire, partant des pores ou des ouvertures, pénètre dans le tissu, pour donner un tissu bio-artificiel vascularisé.

2. Procédé selon la revendication 1, **caractérisé en ce qu'**on utilise en tant que vaisseau un vaisseau d'origine naturelle, provenant de l'homme ou d'un animal.

3. Procédé selon la revendication 1, **caractérisé en ce qu'**on utilise en tant que vaisseau un vaisseau artificiel.

4. Procédé selon la revendication 3, **caractérisé en ce que** le vaisseau artificiel est constitué d'un polymère biologiquement compatible.

5. Procédé selon l'une des revendications 1 à 4, **caractérisé en ce que** le vaisseau poreux a été ponctuellement préparé avec une substance ou un produit déclenchant un stimulus pour le bourgeonnement vasculaire.

6. Procédé selon l'une des revendications 1 à 5, **caractérisé en ce qu'**on ajoute au milieu de culture des substances favorisant l'angiogenèse.

7. Procédé selon la revendication 6, **caractérisé en ce qu'**on ajoute au milieu de culture des facteurs de croissance.

8. Procédé selon la revendication 7, **caractérisé en ce qu'**on ajoute au milieu de culture, en tant que facteur de croissance, du VEGF (facteur de croissance endothéliale vasculaire).

9. Procédé selon l'une des revendications 1 à 8, **caractérisé en ce que** la perfusion est réalisée sous pression.

10. Procédé selon l'une des revendications 1 à 9, **caractérisé en ce que** la perfusion est réalisée d'une manière pulsatile.

11. Procédé selon l'une des revendications 1 à 10, **caractérisé en ce que** le vaisseau est serré entre la conduite d'amenée et la conduite d'évacuation nécessaire à la perfusion, de façon qu'il soit simultanément positionné et fixé par rapport au tissu à cultiver.

12. Procédé selon l'une des revendications 1 à 11, **caractérisé en ce qu'**on utilise en tant que cellules à l'intérieur du tissu tridimensionnel à vasculariser des cardiomyocytes, et que le tissu obtenu est un tissu cardiaque bio-artificiel.

13. Procédé selon l'une des revendications 1 à 12, **caractérisé en ce qu'**on utilise en tant que cellules des kératinocytes, et que le tissu obtenu est un tissu cutané bio-artificiel.

14. Tissu bio-artificiel vascularisé obtenu selon l'une des revendications 1 à 13, **caractérisé en ce qu'**il est traversé par au moins un vaisseau d'origine naturelle, provenant de l'homme ou d'un animal, ou au moins un vaisseau artificiel biocompatible, d'autres vaisseaux, qui ont bourgeonné dans le tissu, partant du vaisseau.

15. Tissu bio-artificiel vascularisé selon la revendication 14, **caractérisé en ce que** le tissu est un tissu cardiaque artificiel, qui comprend des cardiomyocytes dans une matrice.

16. Tissu bio-artificiel vascularisé selon la revendication 14, **caractérisé en ce que** le tissu est un tissu cutané artificiel, qui comprend des keratinocytes dans une matrice.

17. Réacteur d'essai pour la fabrication contrôlée d'un tissu bio-artificiel par un procédé selon l'une des revendications 1 à 13, **caractérisé par** au moins une chambre de culture de tissu, au moins une entrée et une sortie pour cette chambre/chaque chambre, au moins une entrée et une sortie étant configurées comme des moyens pour supporter un vaisseau, ainsi que par deux parois de délimitation opposées, planes, parallèles et optiquement transparentes, de cette/chaque chambre de culture de tissu, l'une des parois de délimitation planes, optiquement transparentes, de la chambre étant configurée comme un couvercle, et des points d'addition supplémentaires étant, dans le couvercle, disposés au-dessus de chaque chambre.

18. Réacteur d'essai selon la revendications 17, **caractérisé en ce qu'**au moins une entrée et une sortie pouvant être verrouillée sont présentes pour chaque chambre.

19. Réacteur d'essai selon la revendication 18, **caractérisé en ce que** les moyens de verrouillage des entrées et sorties pouvant être verrouillées comprennent des robinets, des vannes, des pinces pour flexible et/ou des diaphragmes.

20. Réacteur d'essai selon l'une des revendications 17 à 19, **caractérisé en ce que** des connexions électriques et/ou des composants électriques sont prévus dans au moins une chambre de culture de tissu.
